# EUROPÄISCHE PATENTSCHRIFT

(11) **EP 1 997 452 B1**
(45) Veröffentlichungstag und Bekanntmachung des Hinweises auf die Patenterteilung: **24.09.2014**
(21) Anmeldenummer: 08009824.7
(22) Anmeldetag: 29.05.2008
(51) Int. Cl.: A61B 19/08, A61B 1/00, H02G 11/02

(54) **Vorrichtung zur sterilen Umhüllung eines sterilisationsempfindlichen Bedienteils**
Device for providing a sterile wrapping for a sterilisation sensitive control
Dispositif d'enveloppement stérile d'un élément de commande sensible à la stérilisation

(30) Priorität: 31.05.2007 DE 102007026235
(43) Veröffentlichungstag der Anmeldung: 03.12.2008
(73) Patentinhaber: Karl Storz GmbH & Co. KG, 78532 Tuttlingen (DE)
(72) Erfinder: Irion, Klaus M., Dr.-Ing., 78576 Emmingen-Liptingen (DE); Graf, Christian, 78576 Emmingen-Liptingen (DE); Schwarz, Peter, 78532 Tuttlingen (DE)
(74) Vertreter: Weller, Wolfgang

(56) Entgegenhaltungen:
- DE-T2- 60 017 242
- DE-U1- 8 812 027
- DE-U1-202007 014 102
- US-A- 5 301 657

## Beschreibung

Die Erfindung betrifft eine Vorrichtung zur sterilen Umhüllung eines sterilisationsempfindlichen Bedienteils, das für einen chirurgischen Eingriff mit einem medizinschen Instrument verbindbar ist, mit einem Handhabungselement, das an einem distalen Ende einen zusammengerafften sterilen Schlauch aufweist, wobei ein medizinisches Instrument an seinem proximalen Endbereich mit einem distalen Ende des Schlauches fest verbunden ist, und wobei eine Übergabestation, in der das sterilisationsempfindliche Bedienteil aufgenommen ist, vorhanden ist, wobei das Handhabungselement mit der Übergabestation derart verbindbar ist, dass das medizinische Instrument und das sterilisationsempfindliche Bedienteil miteinander verbindbar sind, und wobei dieser Zusammenbau von der Übergabestation abziehbar ist, wobei der geraffte Schlauch über das sterilisationsempfindliche Bedienteil ziehbar ist.

Eine solche Vorrichtung ist aus der US 5,301,657 A bekannt. Diese Vorrichtung zur sterilen Umhüllung eines Kameraelements, das für einen chirurgischen Eingriff mit einer Sonde verbindbar ist, weist einen zusammengerafften sterilen Schlauch auf. Dieser sterile Schlauch kann mit Hilfe einer Lasche nach Verbinden der Vorrichtung mit dem Kameraelement in proximale Richtung über dieses Kameraelement gezogen werden. Wahlweise kann das Kameraelement hierfür in einen separaten Ständer aufgenommen werden, der dieses Kameraelement beim Zusammenbringen mit der Vorrichtung und dem Überziehen des sterilen Schlauchs hält.

Eine weitere Vorrichtung zur sterilen Umhüllung eines sterilisationsempfindlichen Bedienteils ist aus der DE 39 20 508 A1 bekannt.

Solche Vorrichtungen kommen bei chirurgischen Eingriffen zum Einsatz, bei denen Gerätschaften verwendet werden, die nicht oder nur schwer sterilisierbar sind.

Zu solchen sterilisationsempfindlichen Bedienteilen gehören Kamerasysteme, die in Verbindung mit den Endoskopen bei unterschiedlichen chirurgischen Eingriffen verwendet werden.

Ein endoskopisches Kamerasystem beinhaltet einen Kamerakopf, der empfindliche innere elektronische Bauteile aufweist, z.B. eine Signalprozesseinheit, die die Signale von einer Bilderfassungsoptik in Videosignale verarbeitet, die geeignet sind, einem Monitor zugeführt zu werden.

Solch ein Kamerasystem wird vor einem chirurgischen Eingriff mit einem medizinischen Instrument, z.B. einem Endoskop, verbunden. Während des chirurgischen Eingriffes kann der Operateur die Operationsstelle an einem Monitor beobachten. Der Chirurg handhabt den Zusammenbau über ein Bedienteil des Kamerasystems. An dem Bedienteil können noch andere Steuerelemente, wie Schalter, Hähne, elektrische Stecksysteme etc. vorhanden sein, über die zusätzliche Gerätschaften des Instrumentes, wie z.B. Saug- und Spülleitungen bedient werden können. Dieses Bedienteil ist aufgrund seiner Komplexität und der empfindlichen Bauteile sterilisationsempfindlich.

Um eine Sterilisation des Kamerasystems bzw. des proximalen Handhabungsteils zu vermeiden, wird das Kamerasystem und sein Anschlusskabel, bevor es auf das Endoskop aufgesetzt wird, mit einem sterilen Überzug in Form eines Schlauches überzogen. Nach dem Eingriff wird das Kamerasystem von dem Endoskop getrennt.

Die aus der DE 3 920 508 A1 bekannte Vorrichtung weist dazu ein Handhabungselement in Form eines Aufnehmers auf, an dem ein zusammengeraffter steriler Überzug fixiert ist. Ein Bedienteil einer sterilisationsempfindlichen Kamera wird durch eine Öffnung in dem Aufnehmer so weit in den Aufnehmer eingeführt, bis die Kamera in dem Aufnehmer fixiert ist. Dann wird von einer sterilen Person eine äußere Schutzhülle, mit der der Aufnehmer überzogen ist, geöffnet, und die Kamera wird durch den sterilen Überzug hindurch von einer Hand des Operateurs ergriffen und von dem Aufnehmer weggezogen. Bei der Entnahme der Kamera aus dem Aufnehmer wird die Kamera und das Kamerakabel automatisch mit dem sterilen Überzug umhüllt.

Vor der Entnahme der Kamera aus dem Aufnehmer kann die Kamera mit einem medizinischen Instrument verbunden werden. Dazu weist der sterile Überzug an seinem distalen Ende eine Öffnung auf.

Um das medizinische Instrument mit der sterilisationsempfindlichen Kamera, die in dem Aufnehmer fixiert ist, zu verbinden, wird ein proximales Ende des medizinischen Instruments durch die distale Öffnung in dem sterilen Überzug in das Innere des Überzugs eingeführt und dort mit einem Kupplungselement der sterilisationsempfindlichen Kamera verbunden.

Das Durchfädeln bzw. das Durchschieben des Instruments durch die enge Öffnung erfordert eine hohe Aufmerksamkeit und eine gewisse Geschicklichkeit. Üblicherweise werden dabei beide Hände einer Person benötigt, oder eine Person hält den Aufnehmer und die bereits in den Überzug eingeschobene Kamera und eine andere Person führt von der anderen Seite her das Instrument durch die enge Öffnung in den Überzug ein und koppelt dieses mit der Kamera. Diese Vorgehensweise ist umständlich und verlängert unnötig die Vorbereitungszeit für einen chirurgischen Eingriff. Ferner können über die distale Öffnung Kontaminationen in den Innenraum des sterilen Überzuges gelangen und somit an das eigentlich zu schützende Bedienteil der Kamera gelangen.

Ferner erweist es sich als Nachteil, dass bei der Entnahme der Kamera aus dem Aufnehmer eine sterile Person die Kamera durch den schlauchförmigen Überzug hindurch ergreifen muss. Dabei kann auch der Überzug beeinträchtigt werden. Dies könnte dazu führen, dass die sterilisationsempfindliche Kamera dennoch kontaminiert wird.

Der Erfindung liegt daher die Aufgabe zugrunde, eine Vorrichtung zur sterilen Umhüllung eines sterilisationsempfindlichen Bedienteils der eingangs genannten Art dahingehend weiterzuentwickeln, dass das Verbinden des medizinischen Instrumentes mit dem sterilisationsempfindlichen Bedienteil und das Umhüllen des sterilisationsempfindlichen Bedienteils einfach, möglichst sogar einhändig, durch eine Person durchgeführt werden kann, ohne dass die Gefahr der Kontaminierung des sterilisationsempfindlichen Bedienteils besteht.

Erfindungsgemäß wird die Aufgabe dadurch gelöst, dass das Handhabungselement ferner eine Öffnung aufweist, durch die von proximal nach distal das sterilisationsempfindliche Bedienteil in den sterilen Schlauch derart einführbar ist, dass dabei das sterilisationsempfindliche Bedienteil mit dem sterilen Schlauch umhüllbar ist, dass das Handhabungselement distalseitig eine Handhabe aufweist, und dass die Übergabestation einen An- und Abdockmechanismus aufweist, der über die Handhabe betätigbar ist.

Das feste Verbinden des medizinischen Instrumentes mit dem distalen Ende des mit dem Handhabungselement verbundenen sterilen Schlauches hat zunächst den Vorteil, dass diese drei Bauelemente als kompakte sterile Baueinheit vorgefertigt, zusammengebaut und sterilisiert werden können. Ferner hat die vorgefertigte Baueinheit den erheblichen Vorteil, dass in nur einem Schritt sowohl das medizinische Instrument mit dem sterilisationsempfindlichen Bedienteil verbunden wird, als auch der sterile Schlauch in Bezug auf die Übergabestation derart platziert wird, dass das sterilisationsempfindliche Bedienteil bei dessen Herausziehen aus der Übergabestation automatisch mit dem Schlauch umhüllt wird.

Dieser Schritt ist mit einer (der sterilen) Hand einer Person vom sterilen Bereich aus durchführbar, ohne dass die Gefahr besteht, dass der sterile Bereich kontaminiert wird oder dass der Schlauch beeinträchtigt wird. Mit der anderen (der nicht zwingend sterilen) Hand kann diese Person zuvor die Übergabestation vorbereiten.

Bei einer Vorgehensweise wird zuerst das sterilisationsempfindliche Bedienteil im nichtsterilen Bereich in der Übergabestation fixiert. Im sterilen Bereich wird die sterile Baueinheit aus Handhabungselement, Schlauch und dem medizinischen Instrument von der Person mit einer Hand an die Übergabestation herangebracht und mit der Übergabestation verbunden. Dabei wird gleichzeitig das medizinische Instrument mit dem in der Übergabestation aufgenommenen sterilisationsempfindlichen Bedienteil verbunden.

Das sterile medizinische Instrument kann jetzt vom Operateur ergriffen werden. Durch Abziehen des medizinischen Instruments von der Übergabestation wird das mit diesem zwischenzeitlich verbundene sterilisationsempfindliche Bedienteil aus der Übergabestation herausgezogen und dabei automatisch mit dem sterilen Schlauch umhüllt.

Das medizinische Instrument kann vielseitig ausgestaltet sein. Es kann ein Endoskop sein das reine Beobachtungsfunktion ausübt, es kann auch zusätzlich oder ausschließlich ein Werkzeug also ein arbeitendes Instrument sein. Dazu gehören bspw. Werkzeuge zur Gewebemanipulation, wie Bohrer oder Schneideelemente. Diese können motorisch angetrieben sein. Der Antrieb bzw. der Motor sind im Bedienteil aufnehmbar.

Die Maßnahme, dass das Handhabungselement distalseitig eine Handhabe aufweist, hat den Vorteil, dass durch die Handhabe der Andockvorgang der Baueinheit an der Übergabestation besonders sicher und einfach mit einer (der sterilen) Hand durchgeführt werden kann.

Die Maßnahme, dass die Übergabestation einen An- und Abdockmechanismus aufweist, der über die Handhabe betätigbar ist, hat den Vorteil, dass der An- und Abdockmechanismus von dem sterilen Bereich aus betätigt werden kann. Somit kann die Betätigung des An- und Abdockmechanismus ebenfalls einhändig von derselben Person, die den Zusammenbau aus dem Handhabungselement und dem medizinischem Instrument an die Übergabestation gebracht hat, durchgeführt werden.

In einer weiteren Ausgestaltung der Erfindung weist das Handhabungselement proximalseitig eine Kupplung auf.

Diese Maßnahme hat den Vorteil, dass die Kupplung, die an die nicht sterilisierte Übergabestation gebracht wird, um das Handhabungselement mit der Übergabestation zu verbinden, von dem sterilen Bereich, in dem sich sowohl die Handhabe als auch der sterile Schlauch und das mit dem Schlauch fest verbundene medizinische Instrument befinden, räumlich getrennt ist. Dies trägt weiter dazu bei, dass der sterile Bereich nicht kontaminiert wird.

In einer weiteren Ausgestaltung der Erfindung weist das Handhabungselement ein flächiges Basisteil auf, von dem die Handhabe und die Kupplung vorspringen.

Diese Maßnahme hat den Vorteil, dass das flächige Basisteil die Handhabe und die Kupplung voneinander trennt. Dadurch wird dazu beigetragen, dass der Operateur die Kupplung nicht versehentlich ergreift. Das Basisteil verhindert, dass die Hand, die die Handhabe erfasst hat mit der Übergabestation in Berührung tritt.

In einer weiteren Ausgestaltung der Erfindung ist der sterile Schlauch an der Handhabe befestigt.

Diese Maßnahme hat den Vorteil, dass der geraffte Schlauch in einer kompakten Bauweise angebracht werden kann.

In einer weiteren Ausgestaltung der Erfindung ist die Handhabe als Ringflansch ausgebildet.

Diese Maßnahme hat den Vorteil, dass eine derartig ausgebildete Handhabe sicher von einem Operateur gehalten werden kann. Dadurch wird sichergestellt, dass das Handhabungselement mit dem sterilen Schlauch und dem medizinischen Instrument beim Anbringen dessen an die Übergabestation dem Operateur nicht aus der Hand fällt.

In einer weiteren Ausgestaltung der Erfindung weist die Kupplung ein Kupplungselement auf.

Diese Maßnahme hat den Vorteil, dass das Handhabungselement einfach und gezielt mit der Übergabestation verbindbar ist.

In einer weiteren Ausgestaltung der Erfindung ist die Kupplung als Bajonettkupplung ausgebildet.

Diese Maßnahme hat den Vorteil, die Bajonettkupplung konstruktiv einfach ausgestaltet sein kann. Das Kupplungselement an dem Handhabungselement kann als ein Stift ausgebildet werden, der durch einen fertigungstechnisch äußerst einfachen Vorgang bewerkstelligt werden kann. Eine Bajonettführung an der Übergabestation kann durch einen einfachen Einfräsvorgang bewerkstelligt werden.

In einer weiteren Ausgestaltung der Erfindung weist das Basisteil des Handhabungselements ein Orientierungsmerkmal auf.

Diese Maßnahme hat den Vorteil, dass durch Vorsehen des Orientierungsmerkmals an dem Basisteil der Operateur besonders einfach erkennen kann, in welcher Stellung das Handhabungselement zum Andocken an die Übergabestation gebracht werden muss.

In einer weiteren Ausgestaltung der Erfindung sind das Handhabungselement und der Schlauch als Einwegelemente ausgebildet.

Diese Maßnahme hat den Vorteil, dass nach dem Einsatz das Handhabungselement und der Schlauch nicht gereinigt werden müssen, sondern entsorgt werden können.

In einer weiteren Ausgestaltung der Erfindung ist das medizinische Instrument als Einweginstrument ausgebildet.

Auch hierbei besteht wiederum der Vorteil, dass nach dem Einsatz das medizinische Instrument nicht sterilisiert werden muss, sondern zusammen mit dem Handhabungselement und dem Schlauch entsorgt werden kann.

In einer weiteren Ausgestaltung der Erfindung, die alternativ zu der zuvor genannten Ausgestaltung verwendet werden kann, ist das medizinische Instrument als Mehrweginstrument ausgebildet.

Diese Maßnahme ist für teure Instrumente vorteilhaft. Bei dieser Ausgestaltung muss das medizinische Instrument nach Gebrauch von dem Schlauch und dem Handhabungselement getrennt werden und kann gereinigt und sterilisiert werden, um danach wieder mit dem Schlauch eines neuen Handhabungselements verbunden zu werden.

In einer weiteren Ausgestaltung der Erfindung ist der An- und Abdockmechanismus über Bedienelemente, die an der Übergabestation angeordnet sind, betätigbar.

Bei dieser Ausgestaltung müssen Bedienelemente, die an der nicht sterilisierten Übergabestation angeordnet sind, vor Gebrauch mit einer sterilen Abdeckung versehen werden. Das Betätigen des An- und Abdockmechanismus mit den Bedienelementen kann auf mechanische oder elektronische Weise erfolgen.

In einer weiteren Ausgestaltung der Erfindung weist der An- und Abdockmechanismus eine Ausgangsstellung auf, in der das sterilisationsempfindliche Bedienteil in der Übergabestation verriegelt ist.

Diese Maßnahme hat den Vorteil, dass das sterilisationsempfindliche Bedienteil in der Übergabestation gegen Herausziehen, Hineinschieben und Verdrehen verriegelt ist. Somit kann der Operateur mit einer Hand den Zusammenbau bestehend aus dem Handhabungselement mit dem medizinischen Instrument an die Übergabestation bringen und das medizinische Instrument mit dem sterilisationsempfindlichen Bedienteil verbinden, ohne dass er die Übergabestation bzw. das sterilisationsempfindliche Bedienteil ergreifen muss.

In einer weiteren Ausgestaltung der Erfindung weist der An- und Abdockmechanismus eine erste Stellung auf, in der das Handhabungselement an der Übergabestation verriegelt ist und das sterilisationsempfindliche Bedienteil und das damit verbundene medizinische Instrument zum Herausziehen freigegeben sind.

Diese Maßnahme hat den Vorteil, dass erst nachdem das Handhabungselement an der Übergabestation verriegelt ist, das sterilisationsempfindliche Bedienteil und das medizinische Instrument zum Herausziehen freigegeben werden. Dadurch wird sichergestellt, dass das Bedienteil erst dann aus der Übergabestation herausgezogen werden kann, wenn der sterile an dem Handhabungselement angeordnete Schlauch so platziert ist, dass das sterilisationsempfindliche Bedienteil beim Herausziehen aus der Übergabestation automatisch mit dem sterilen Schlauch umhüllt wird.

In einer weiteren Ausgestaltung der Erfindung weist der An- und Abdockmechanismus eine zweite Stellung auf, in der das sterilisationsempfindliche Bedienteil und das damit verbundene medizinische Instrument zum Rückführen freigegeben sind.

Diese Maßnahme hat zum einen den Vorteil, dass wenn das sterilisationsempfindliche Bedienteil, das üblicherweise ein Kabel aufweist, zu weit herausgezogen wird, über kurzzeitige Betätigung der Handhabe (von der ersten Stellung auf die zweite Stellung des An- und Abdockmechanismus) das Bedienteil mit dem Kabel zur Rückführung freigegeben wird. Zum anderen ermöglicht eine derartige Ausgestaltung des An- und Abdockmechanismus, dass das sterilisationsempfindliche Bedienteil nach erfolgter Arbeit wieder in die Übergabestation zurückgeführt werde kann. Das Rückführen des sterilisationsempfindlichen Bedienteils in die Übergabestation kann über einen Federmechanismus oder elektromotorisch erfolgen.

In einer weiteren Ausgestaltung der Erfindung weist der An- und Abdockmechanismus eine dritte Stellung auf, in der die Verbindung zwischen dem medizinischen Instrument und dem sterilisationsempfindlichen Bedienteil lösbar ist.

Diese Maßnahme hat den Vorteil, dass nach dem Einsatz das medizinische Instrument von dem in der Übergabestation aufgenommenen sterilisationsempfindlichen Bedienteil getrennt werden kann.

In einer weiteren Ausgestaltung der Erfindung weist der An- und Abdockmechanismus eine vierte Stellung auf, in der die Verriegelung des Handhabungselements lösbar ist.

Diese Maßnahme hat den Vorteil, dass das Handhabungselement von der Übergabestation gelöst werden kann.

Weitere Merkmale und Vorteile ergeben sich aus der nachfolgenden Beschreibung und der beiliegenden Zeichnung.

Es versteht sich, dass die vorstehend genannten und die nachstehend noch zu erläuternden Merkmale nicht nur in der angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand ausgewählter Ausführungsbeispiele in Zusammenhang mit der beiliegenden Zeichnung näher beschrieben und erläutert. Es zeigen:
- Figur 1: eine perspektivische Ansicht einer erfindungsgemäßen Vorrichtung;
- Figur 2: eine vergrößerte perspektivische Ansicht des distalen Bereichs eines Handhabungselements, das mit dem medizinischen Instrument verbunden ist;
- Figur 3: eine perspektivische Ansicht des proximalen Bereichs des Hand-habungselements;
- Figur 4: eine Situation, in der das Handhabungselement mit der Übergabestation und das medizinische Instrument mit dem sterilisationsempfindlichen Bedienteil verbunden ist; und
- Figur 5: eine der Darstellung in Figur 4 vergleichbare Darstellung, wobei das sterilisationsempfindliche Bedienteil von der Übergabestation abgezogen ist und mit dem sterilen Schlauch umhüllt ist.

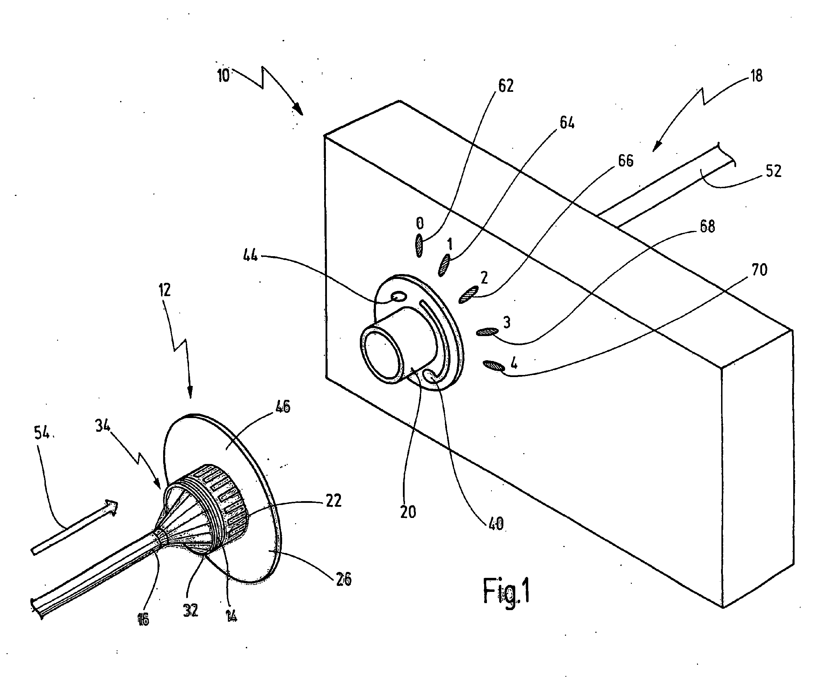

Eine in Figuren 1 bis 5 dargestellte Vorrichtung zur sterilen Umhüllung eines sterilisationsempfindlichen Bedienteils ist in ihrer Gesamtheit mit der Bezugsziffer 10 versehen.

Die in Figur 1 dargestellte erfindungsgemäße Vorrichtung 10 weist ein Handhabungselement 12 auf, das einen zusammengerafften sterilen Schlauch 14 aufweist. Mit dem Schlauch 14 ist ein medizinisches Instrument 16 fest verbunden.

Ferner weist die erfindungsgemäße Vorrichtung 10 eine Übergabestation 18 auf, in der ein sterilisationsempfindliches Bedienteil 20, das hier nur fragmentarisch dargestellt ist, aufgenommen ist.

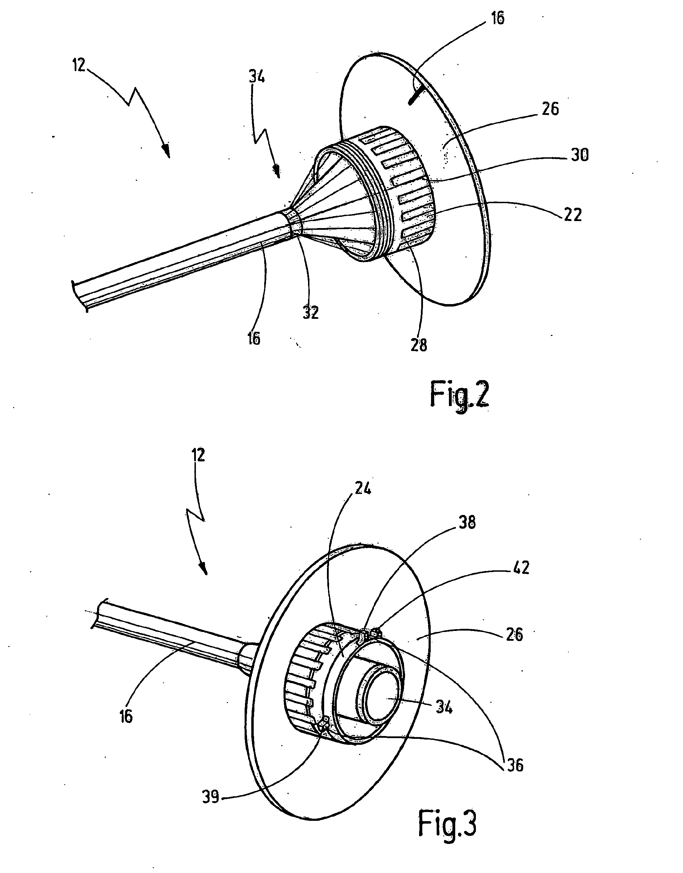

Das Handhabungselement 12 weist distalseitig eine Handhabe 22 auf, wie es insbesondere aus der vergrößerten Darstellung in Figur 2 ersichtlich ist.

Proximalseitig weist das Handhabungselement 12 eine Kupplung 24 auf, die aus der Darstellung in Figur 3 ersichtlich ist. Mittels der Kupplung 24 wird das Handhabungselement 12 mit der Übergabestation 18 verbunden.

Ferner weist das Handhabungselement 12 ein flächiges Basisteil 26 auf, von dem die Handhabe 22 und die Kupplung 24 vorspringen. In diesem Ausführungsbeispiel ist das Basisteil 26 scheibenförmig ausgebildet. Die Größe des Basisteils 26 ist so gewählt, dass eine Hand, die die Handhabe 22 ergriffen hat, beim Ankoppeln an die Übergabestation 18 mit dieser nicht in Berührung tritt. Dadurch wird vermieden, dass allfällige Kontamination von der nicht sterilen Übergabestation 18 an das Handhabungselement 12 gelangen. Dementsprechend kann vermieden werden, dass der Arzt, der im Einsatz das Handhabungselement 12 erfasst dadurch kontaminiert wird.

Die Handhabe 22 ist als Ringflansch 28 ausgebildet, in dessen Außenseite ein Kerbenmuster 30 eingeschnitten ist, so dass die Handhabe 22 sicher von Fingern einer menschlichen Hand ergriffen werden kann.

An der Handhabe 22 ist ein proximales Ende des sterilen Schlauchs 14 fixiert. Ein distales Ende des Schlauchs 14, der in Figuren 2 und 3 in einer zusammengerafften Form dargestellt ist, ist an dem medizinischen Instrument 16 fixiert. In diesem Ausführungsbeispiel ist das distale Ende 32 des Schlauchs 14 an einem proximalen Endbereich 34 des medizinischen Instruments 16 fixiert. Die Fixierung erfolgt bei diesem Ausführungsbeispiel durch Ankleben des Schlauchs 14 an dem medizinischen Instrument 16. Das proximale Ende 34 des medizinischen Instrumentes 16 steckt in einer hier nicht ersichtlichen Öffnung im Basisteil 26.

Die proximal angeordnete Kupplung 24, die aus der Darstellung von Figur 3 ersichtlich ist, ist in diesem Ausführungsbeispiel als Bajonettkupplung ausgebildet.

Die als Bajonettkupplung ausgebildete Kupplung 24 weist ein Kupplungselement 36 auf, das Stifte 38, 39 aufweist. An der Übergabestation 18 ist eine Bajonettführung 40 angeordnet, wie es insbesondere aus der Darstellung in Figur 1 ersichtlich ist. Beim Verbinden des Handhabungselements 12 mit der Übergabestation 18 greifen die Stifte 38, 39 in die Bajonettführung 40 ein. Durch Drehen des Handhabungselements 12 kommt es zum Verriegeln.

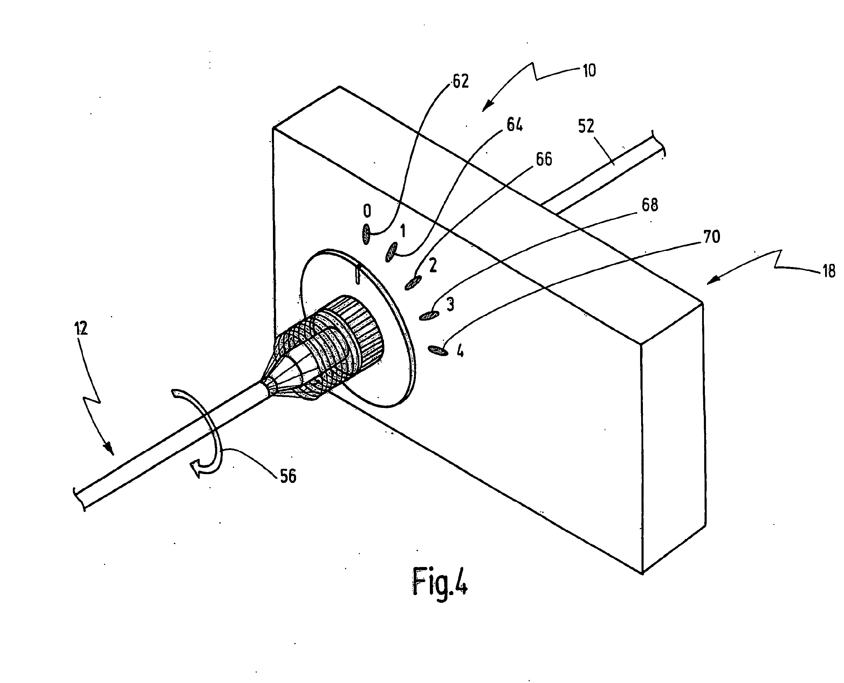

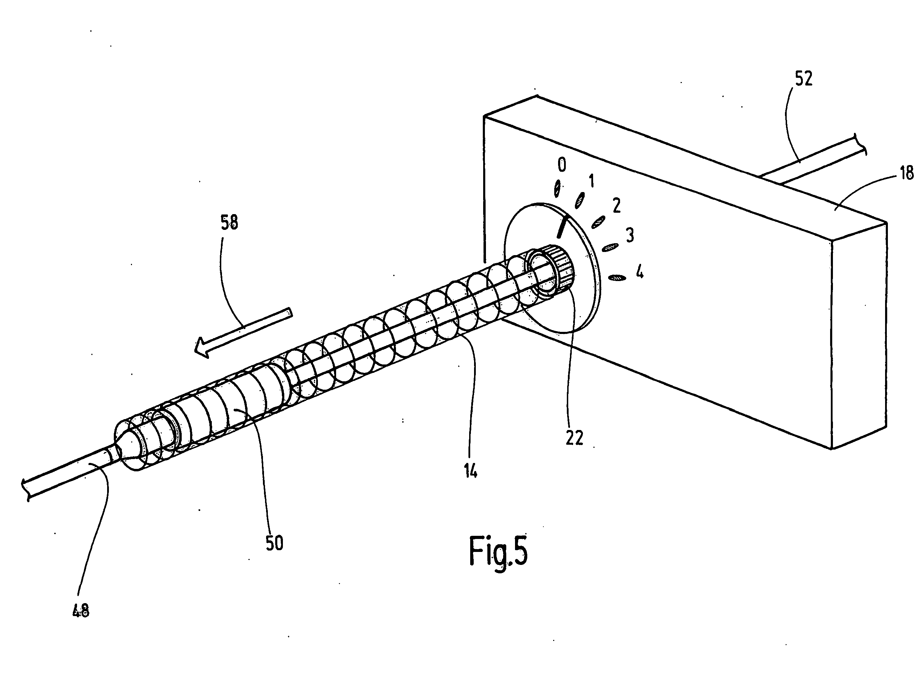

Ferner weist das Handhabungselement 12 eine proximalseitig angeordnete Steuernocke 42 auf. Beim Verbinden des Handhabungselements 12 mit der Übergabestation 18 wird die Steuernocke 42, mit einem Kontaktschalter 44, der an der Übergabestation 18 angeordnet ist, in Verbindung gebracht. Dadurch wird ein An- und Abdockmechanismus 60, der noch später in Zusammenhang mit Figuren 4 und 5 näher beschrieben wird, betätigt.

An dem Basisteil 26 ist ein Orientierungsmerkmal 46 angeordnet, dessen Funktion noch später näher beschrieben wird.

Das medizinische Instrument 16, das mit dem distalen Ende 32 des Schlauchs 14 fest verbunden ist und in den Figuren 1 bis 5 nur fragmentarisch dargestellt ist, ist in diesem Ausführungsbeispiel als Endoskop 48 ausgebildet. Das in diesem Ausführungsbeispiel dargestellte Endoskop 48 ist als Einweginstrument ausgebildet.

Das als Einweginstrument ausgebildete Endoskop 48 weist folgende Elemente auf, die aus keiner der Figuren ersichtlich sind, nämlich zumindest einen distalseitig angeordneten Video-Sensor mit einem Objektiv, ein Lichtübertragungselement, welches Licht von proximal nach distal leitet, und ein elektrisches Übertragungssystem, welches die Bildinformation überträgt.

In diesem Ausführungsbeispiel ist das proximale Ende 34 des Endoskops 48 derart angeordnet, dass es proximal über die Kupplung 24 hinausragt, wie es insbesondere aus der Darstellung von Figur 3 ersichtlich ist.

Das sterilisationsempfindliche Bedienteil 20 ist in diesem Ausführungsbeispiel als ein Handgriff 50 ausgebildet, der mit einem Kabel 52 verbunden ist. Das sterilisationsempfindliche Bedienteil 20 enthält empfindliche elektronische Bauteile und eine integrierte Beleuchtungseinheit mit LEDs bzw. Lichtfasern, die hier nicht dargestellt sind.

Das Bedienteil 20 ist in der Übergabestation 18 derart aufgenommen, dass dessen distales Ende vor der Übergabestation 18 vorsteht, wie es insbesondere aus der Darstellung von Figur 1 ersichtlich ist.

Das distale Ende des Bedienteils 20 ist derart ausgebildet, dass es geeignet ist, das proximale 34 Ende des medizinischen Instruments 16 aufzunehmen, so dass das medizinische Instrument 16 mit dem Bedienteil 20 gekoppelt wird.

Anhand von Figuren 4 und 5 in Verbindung mit Figur 1 soll eine Handhabung der erfindungsgemäßen Vorrichtung 10 erläutert werden.

Das sterilisationsempfindliche Bedienteil 20 wird im nichtsterilen Bereich in der Übergabestation 18 eingeschoben und gegen Herausziehen, Hineinschieben und Verdrehen verriegelt. Solch eine Situation ist in Figur 1 dargestellt.

Ein Zusammenbau bestehend aus dem Handhabungselement 12, dem sterilen Schlauch 14 und dem mit dem sterilen Schlauch 14 fest verbundenen medizinischen Instrument 16 wird vom Operateur im sterilen Bereich aus einer sterilen Verpackung entnommen.

Diese kompakte Baueinheit wird von einer sterilen Hand einer Person an der Handhabe 22 ergriffen und der Zusammenbau wird an die Übergabestation 18 gebracht (siehe Pfeil 54, Figur 1).

Zuerst wird das proximale Ende 34 des medizinischen Instruments 16 in das distale Ende des Bedienteils 20, das vor der Übergabestation 18 vorsteht, eingeführt. Dadurch wird das medizinische Instrument 16 mit dem Bedienteil 20 verbunden.

Gleichzeitig werden die Stifte 38, 39 der Kupplung 24 in die Bajonettführung 40, die an der Übergabestation 18 angeordnet ist, eingeführt. Solch eine Situation ist in Figur 4 dargestellt.

Beim Anbringen des Handhabungselements 12 an die Übergabestation 18 ist das Handhabungselement 12 derart auszurichten, dass sich das Orientierungsmerkmal 46 des Basisteils 26 in Ausrichtung mit einer Ausgangsstellung 62 des An- und Abdockmechanismus 60 befindet.

Diese Vorgänge können mit einer einzigen, der sterilen, Hand einer Person durchgeführt werden.

Durch Drehen der Handhabe 22 von der Ausgangsstellung 62 auf die erste Stellung 64 des An- und Abdockmechanismus 60 wird das Handhabungselement 12 mittels der Bajonettkupplung an der Übergabestation 18 verriegelt (siehe Pfeil 56, Figur 1).

In der ersten Stellung 64 wird gleichzeitig das in der Übergabestation 18 verriegelte Bedienteil 20 zum Herausziehen freigegeben.

In dieser Stellung 64 wird das medizinische Instrument 16, das im sterilen Bereich angeordnet ist, vom Operateur ergriffen und von der Übergabestation abgezogen (siehe Pfeil 58, Figur 5). Dadurch wird das Bedienteil 20 aus der Übergabestation 18 herausgezogen. Beim Herausziehen entfaltet sich der sterile Schlauch 14 automatisch über das Bedienteil 20 und das Kabel 48. Solch eine Situation ist in Figur 5 dargestellt.

In der ersten Stellung 64 des An- und Abdockmechanismus 60, die als Arbeitsstellung bezeichnet wird, ist das medizinische Instrument 16 mit dem Bedienteil 20 nun frei bewegbar.

Wurde das Bedienteil 20 zu weit herausgezogen, kann das Bedienteil 20 über kurzzeitiges Betätigen der Handhabe 22 von der ersten Stellung 64 auf eine zweite Stellung 66 zur Rückführung freigegeben werden.

Nach erfolgtem chirurgischem Eingriff wird das Handhabungselement 12 durch Drehen der Handhabe 22 auf die zweite Stellung 66 des An- und Abdockmechanismus 60 gebracht. In der zweiten Stellung 66 des An- und Abdockmechanismus 60 wird das Bedienteil 20 und damit das Handgriff 48 und das medizinische Instrument 16 zurückgezogen, und zwar so weit bis das Bedienteil 20 in der Übergabestation 18 verriegelt wird. Das Zurückziehen erfolgt über einen Federmechanismus oder elektromotorisch.

Beim Zurückziehen faltet sich der Schlauch 14 mehr oder weniger vor der Handhabe 22 zusammen.

Dann wird das Handhabungselement 12 durch Drehen der Handhabe 22 auf eine dritte Stellung 68 des An- und Abdockmechanismus 60 gebracht. In dieser Stellung 68 wird die Verbindung zwischen dem medizinischen Instrument 16 und dem Bedienteil 20 gelöst, so dass das medizinische Instrument 16 von dem Bedienteil 20 getrennt wird.

Dann wird das Handhabungselement 12 durch Drehen der Handhabe 22 auf eine vierte Stellung 70 des An- und Abdockmechanismus 60 gebracht. In der vierten Stellung 70 wird das Handhabungselement 12 von der Übergabestation 18 gelöst.

Der von der Übergabestation 18 abgenommene Zusammenbau bestehend aus dem Handhabungselement 12, dem Schlauch 14 und dem medizinischen Instrument 16, das bei diesem Ausführungsbeispiel als Einweginstrument ausgebildet ist, wird ohne Reinigung entsorgt.

Auch dieser Vorgang kann mit einer Hand durchgeführt werden.

Wenn ein Mehrweginstrument verwendet wird, ist die Handhabung der erfindungsgemäßen Vorrichtung 10 gleich wie beim Einweginstrument, nur mit dem Unterschied, dass das Mehrweginstrument, bevor es mit dem Schlauch 14 fest verbunden wird, sterilisiert werden muss. Nach erfolgter Arbeit wird das Mehrweginstrument von dem Schlauch 14 getrennt und gereinigt.

## Patentansprüche

1. Vorrichtung zur sterilen Umhüllung eines sterilisationsempfindlichen Bedienteils, das für einen chirurgischen Eingriff mit einem medizinischen Instrument verbindbar ist, mit einem Handhabungselement (12), das an einem distalen Ende einen zusammengerafften sterilen Schlauch (14) aufweist, wobei ein medizinisches Instrument (16) an seinem proximalen Endbereich (34) mit einem distalen Ende (32) des sterilen Schlauchs (14) fest verbunden ist, und wobei eine Übergabestation (18), in der das sterilisationsempfindliche Bedienteil (20) aufgenommen ist, vorhanden ist, wobei das Handhabungselement (12) mit der Übergabestation (18) derart verbindbar ist, dass das medizinische Instrument (16) und das sterilisationsempfindliche Bedienteil (20) miteinander verbindbar sind, und wobei dieser Zusammenbau von der Übergabestation (18) abziehbar ist, wobei der geraffte sterile Schlauch (14) über das sterilisationsempfindliche Bedienteil (20) ziehbar ist, **dadurch gekennzeichnet, dass** das Handhabungselement (12) ferner eine Öffnung aufweist, durch die von proximal nach distal das sterilisationsempfindliche Bedienteil (20) in den sterilen Schlauch (14) derart einführbar ist, dass dabei das sterilisationsempfindliche Bedienteil (20) mit dem sterilen Schlauch (14) umhüllbar ist, dass das Handhabungselement (12) distalseitig eine Handhabe (22) aufweist, und dass die Übergabestation (18) einen An- und Abdockmechanismus (60) für das Handhabungselement (12) aufweist, der über die Handhabe (22) betätigbar ist.

2. Vorrichtung nach Anspruch 1, **dadurch gekennzeichnet, dass** das Handhabungselement (12) proximalseitig eine Kupplung (24) aufweist.

3. Vorrichtung nach Anspruch 1 oder 2, **dadurch gekennzeichnet, dass** das Handhabungselement (12) ein flächiges Basisteil (26) aufweist, von dem die Handhabe (22) und die Kupplung (24) vorspringen.

4. Vorrichtung nach einem der Ansprüche 1 bis 3, **dadurch gekennzeichnet, dass** der sterile Schlauch (14) an der Handhabe (22) befestigt ist.

5. Vorrichtung nach einem der Ansprüche 1 bis 4, **dadurch gekennzeichnet, dass** die Handhabe (22) als Ringflansch (28) ausgebildet ist.

6. Vorrichtung nach einem der Ansprüche 2 bis 5, **dadurch gekennzeichnet, dass** die Kupplung (24) ein Kupplungselement (36) aufweist.

7. Vorrichtung nach einem der Ansprüche 2 bis 6, **dadurch gekennzeichnet, dass** die Kupplung (24) als Bajonettkupplung ausgebildet ist.

8. Vorrichtung nach einem der Ansprüche 3 bis 7, **dadurch gekennzeichnet, dass** das Basisteil (26) ein Orientierungsmerkmal (46) aufweist.

9. Vorrichtung nach einem der Ansprüche 1 bis 8, **dadurch gekennzeichnet, dass** das Handhabungselement (12) und der Schlauch (14) als Einwegelemente ausgebildet sind.

10. Vorrichtung nach einem der Ansprüche 1 bis 9, **dadurch gekennzeichnet, dass** das medizinische Instrument (16) als Einweginstrument ausgebildet ist.

11. Vorrichtung nach einem der Ansprüche 1 bis 9, **dadurch gekennzeichnet, dass** das medizinische Instrument (16) als Mehrweginstrument ausgebildet ist.

12. Vorrichtung nach einem der Ansprüche 1 bis 11, **dadurch gekennzeichnet, dass** der An- und Abdockmechanismus (60) über Bedienelemente, die an der Übergabestation (18) angeordnet sind, betätigbar ist.

13. Vorrichtung nach einem der Ansprüche 1 bis 12, **dadurch gekennzeichnet, dass** der An- und Abdockmechanismus (60) eine Ausgangsstellung (62) aufweist, in der das sterilisationsempfindliche Bedienteil (20) in der Übergabestation (18) verriegelt ist.

14. Vorrichtung nach einem der Ansprüche 1 bis 13, **dadurch gekennzeichnet, dass** der An- und Abdockmechanismus (60) eine erste Stellung (64) aufweist, in der das Handhabungselement (12) an der Übergabestation (18) verriegelt ist und das sterilisationsempfindliche Bedienteil (20) und das damit verbundene medizinische Instrument (16) zum Abziehen von der Übergabestation (18) freigegeben sind.

15. Vorrichtung nach einem der Ansprüche 1 bis 14, **dadurch gekennzeichnet, dass** der An- und Abdockmechanismus (60) eine zweite Stellung (66) aufweist, in der das sterilisationsempfindliche Bedienteil (20) und das damit verbundene medizinische Instrument (16) zum Rückführen zur Übergabestation (18) freigegeben sind.

16. Vorrichtung nach einem der Ansprüche 1 bis 15, **dadurch gekennzeichnet, dass** der An- und Abdockmechanismus (60) eine dritte Stellung (68) aufweist, in der die Verbindung zwischen dem medizinischen Instrument (16) und dem sterilisationsempfindlichen Bedienteil (20) lösbar ist.

17. Vorrichtung nach einem der Ansprüche 1 bis 16, **dadurch gekennzeichnet, dass** der An- und Abdockmechanismus (60) eine vierte Stellung (70) aufweist, in der die Verriegelung des Handhabungselements (12) mit der Übergabestation (18) lösbar ist.

## Claims

1. Device for the sterile sheathing of a sterilization-sensitive operating part, which can be connected to a medical instrument for a surgical intervention, with a handling element (12), which comprises a bundled sterile hose (14) at a distal end, wherein a medical instrument (16) is connected fixedly at its proximal end region (34) to a distal end (32) of the sterile hose (14), and with a transfer station (18), in which the sterilization-sensitive operating part (20) is accommodated, whereby the handling element (12) can be connected to the transfer station (18) such that the medical instrument (16) and the sterilization-sensitive operating part (20) can be connected to one another and wherein this assembly can be withdrawn from the transfer station (18), whereby the bundled sterile hose (14) can be pulled over the sterilization-sensitive operating part (20), **characterized in that** the handling element (12) further comprises an opening, through which the sterilization-sensitive operating part (20) can be inserted into the sterile hose (14) from proximal to distal such, that the sterilization-sensitive operating part (20) can thereby be sheathed with the sterile hose (14), further **in that** the handling element (12) comprises a handle (22) on the distal side, and **in that** the transfer station (18) comprises a docking and undocking mechanism (60), for the handling element (12), which can be actuated via the handle (22).

2. Device of Claim 1, **characterized in that** the handling element (12) comprises a coupling (24) on the proximal side.

3. Device of Claims 1 or 2, **characterized in that** the handling element (12) comprises a flat base part (26), from which the handle (22) and the coupling (24) protrude.

4. Device of anyone of Claims 1 through 3, **characterized in that** the sterile hose (14) is attached to the handle (22).

5. Device of anyone of Claims 1 through 4, **characterized in that** the handle (22) is designed as an annular flange (28).

6. Device of anyone of Claims 2 through 5, **characterized in that** the coupling (24) comprises a coupling element (36).

7. Device of anyone of Claims 2 through 6, **characterized in that** the coupling (24) is designed as a bayonet coupling.

8. Device of anyone of Claims 3 through 7, **characterized in that** the base part (26) comprises an orientation feature (46).

9. Device of anyone of Claims 1 through 8, **characterized in that** the handling element (12) and the hose (14) are designed as disposable elements.

10. Device of anyone of Claims 1 through 9, **characterized in that** the medical instrument (16) is designed as a disposable instrument.

11. Device of anyone of Claims 1 through 9, **characterized in that** the medical instrument (16) is designed as a reusable instrument.

12. Device of anyone of Claims 1 through 11, **characterized in that** the docking and undocking mechanism (16) can be actuated via operational controls arranged on the transfer station (18).

13. Device of anyone of Claims 1 through 12, **characterized in that** the docking and undocking mechanism (16) comprises in a start position (62), in which the the sterilization-sensitive operating part (20) is locked in the transfer station (18).

14. Device of anyone of Claims 1 through 13, **characterized in that** the docking and undocking mechanism (16) comprises a first position (64) in which the handling element (12) is locked on the transfer station (18), and **in that** the sterilization-sensitive operating part (20) and the medical instrument (16) connected therewith are released for withdrawal from the transfer station.

15. Device of anyone of Claims 1 through 14, **characterized in that** the docking and undocking mechanism (16) comprises a second position (66), in which the sterilization-sensitive operating part (20) and the medical instrument (16) connected therewith are released for return to the transfer station (18).

16. Device of anyone of Claims 1 through 15, **characterized in that** the docking and undocking mechanism (16) comprises a third position (68), in which the connection between the medical instrument (16) and the sterilization-sensitive operating part (20) is disconnectable.

17. Device of anyone of Claims 1 through 16, **characterized in that** the docking and undocking mechanism (60) comprises a fourth position (70), in which the locking of the handling element (12) with the transfer station (18) is unlockable.

## Revendications

1. Dispositif d'enveloppement stérile d'un organe de commande sensible à la stérilisation, lequel peut être combiné pour un contact chirurgical avec un appareil médical, à un élément de manipulation (12), lequel présente au niveau d'une extrémité distale un tuyau souple stérile replié sur lui-même (14), dans lequel un appareil médical (16) est fixé fermement sur sa partie d'extrémité proximale (34) à une extrémité distale (32) du tuyau souple stérile (14), et dans lequel un poste de transfert (18), dans lequel l'organe de commande sensible à la stérilisation (20) est reçu, est disponible, dans lequel l'élément de manipulation (12) peut être combiné au poste de transfert (18) de telle sorte que l'appareil médical (16) et l'organe de commande sensible à la stérilisation (20) peuvent être combinés ensemble, et dans lequel cet assemblage peut être retiré du poste de transfert (18), dans lequel le tuyau souple stérile replié sur lui-même (14) peut être étiré sur l'organe de commande sensible à la stérilisation (20), **caractérisé en ce que** l'élément de manipulation (12) présente en outre une ouverture, à travers laquelle l'organe de commande sensible à la stérilisation (20) peut être inséré dans le tuyau souple stérile (14) depuis l'extrémité proximale vers l'extrémité distale de telle sorte que l'organe de commande sensible à la stérilisation (20) peut être enveloppé avec le tuyau souple stérile (14), **en ce que** l'élément de manipulation (12) présente au niveau d'une extrémité distale une prise (22), et **en ce que** le poste de transfert (18) présente un mécanisme d'arrimage et de désarrimage (60) destiné à l'élément de manipulation (12), lequel mécanisme peut être actionné grâce à la prise (22).

2. Dispositif selon la revendication 1, **caractérisé en ce que** l'élément de manipulation (12) présente au niveau de son extrémité proximale un dispositif d'accouplement (24).

3. Dispositif selon la revendication 1 ou 2, **caractérisé en ce que** l'élément de manipulation (12) présente une partie de base plane (26), de laquelle la prise (22) et le dispositif d'accouplement (24) font saillie.

4. Dispositif selon l'une des revendications 1 à 3, **caractérisé en ce que** le tuyau souple stérile (14) est fixé sur la prise (22).

5. Dispositif selon l'une des revendications 1 à 4, **caractérisé en ce que** la prise (22) est conçue sous la forme d'une collerette annulaire (28).

6. Dispositif selon l'une des revendications 2 à 5, **caractérisé en ce que** le dispositif d'accouplement (24) présente un élément d'accouplement (36).

7. Dispositif selon l'une des revendications 2 à 6, **caractérisé en ce que** le dispositif d'accouplement (24) est conçu sous la forme d'un dispositif d'accouplement à baïonnette.

8. Dispositif selon l'une des revendications 3 à 7, **caractérisé en ce que** la partie de base (26) présente un repère distinctif d'orientation (46).

9. Dispositif selon l'une des revendications 1 à 8, **caractérisé en ce que** l'élément de manipulation (12) et le tuyau souple (14) sont conçus sous la forme d'éléments à voie unique.

10. Dispositif selon l'une des revendications 1 à 9, **caractérisé en ce que** l'appareil médical (16) est conçu sous la forme d'un appareil à voie unique.

11. Dispositif selon l'une des revendications 1 à 9, **caractérisé en ce que** l'appareil médical (16) est conçu sous la forme d'un appareil à plusieurs voies.

12. Dispositif selon l'une des revendications 1 à 11, **caractérisé en ce que** le mécanisme d'amarrage et de désamarrage (60) peut être actionné sur des éléments de commande, lesquels sont agencés sur le poste de transfert (18).

13. Dispositif selon l'une des revendications 1 à 12, **caractérisé en ce que** le mécanisme d'amarrage et de désamarrage (60) présente une position de départ (62), dans laquelle l'organe de commande sensible à la stérilisation (20) est bloqué dans le poste de transfert (18).

14. Dispositif selon l'une des revendications 1 à 13, **caractérisé en ce que** le mécanisme d'amarrage et de désamarrage (60) présente une première position (64), dans laquelle l'élément de manipulation (12) est bloqué sur le poste de transfert (18) et l'organe de commande sensible à la stérilisation (20) et l'appareil médical (16) combiné à ce dernier sont libérés du poste de transfert (18) en vue d'être retirés.

15. Dispositif selon l'une des revendications 1 à 14, **caractérisé en ce que** le mécanisme d'amarrage et de désamarrage (60) présente une deuxième position (66), dans laquelle l'organe de commande sensible à la stérilisation (20) et l'appareil médical (16) combiné à ce dernier sont libérés du poste de transfert (18) en vue d'être enlevés.

16. Dispositif selon l'une des revendications 1 à 15, **caractérisé en ce que** le mécanisme d'amarrage et de désamarrage (60) présente une troisième position (68), dans laquelle la combinaison de l'appareil médical (16) et de l'organe de commande sensible à la stérilisation (20) peut être libérée.

17. Dispositif selon l'une des revendications 1 à 16, **caractérisé en ce que** le mécanisme d'amarrage et de désamarrage (60) présente une quatrième position (70), dans laquelle le blocage de l'élément de manipulation (12) avec le poste de transfert (18) peut être libéré.
